(19) Europäisches Patentamt / European Patent Office / Office européen des brevets

(11) EP 2 694 015 B1

(12) **EUROPEAN PATENT SPECIFICATION**

(45) Date of publication and mention
of the grant of the patent:
**19.07.2017 Bulletin 2017/29**

(21) Application number: **12711644.0**

(22) Date of filing: **02.04.2012**

(51) Int Cl.:
*A61Q 19/00* (2006.01)     *A61K 8/02* (2006.01)
*A61K 8/06* (2006.01)     *A61K 8/19* (2006.01)
*A61K 8/25* (2006.01)     *A61K 8/26* (2006.01)
*A61K 8/27* (2006.01)     *A61K 8/29* (2006.01)
*A61K 8/58* (2006.01)     *A61K 8/73* (2006.01)
*A61K 8/81* (2006.01)     *A61Q 17/04* (2006.01)
*A61Q 1/02* (2006.01)     *A61Q 1/10* (2006.01)
*A61K 8/04* (2006.01)     *A61K 8/28* (2006.01)

(86) International application number:
**PCT/EP2012/055921**

(87) International publication number:
**WO 2012/136607 (11.10.2012 Gazette 2012/41)**

(54) **GRAFTED PARTICLES FOR USE IN SKIN CARE APPLICATIONS**

GEPFROPFTE PARTIKEL ZUR VERWENDUNG IN HAUTPFLEGEANWENDUNGEN

PARTICULES GREFFÉES POUR UNE UTILISATION DANS DES APPLICATIONS DE SOINS DE LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.04.2011 US 201161471877 P
04.07.2011 EP 11172466**

(43) Date of publication of application:
**12.02.2014 Bulletin 2014/07**

(73) Proprietor: **Akzo Nobel Chemicals International
B.V.
6824 BM Arnhem (NL)**

(72) Inventors:
• **LEBLANC, Jean-Pierre
Hillsborough, New Jersey 08844 (US)**
• **MILLER, James Albert
Manville, New Jersey 08873 (US)**

(74) Representative: **Akzo Nobel IP Department
Velperweg 76
6824 BM Arnhem (NL)**

(56) References cited:
EP-A1- 2 107 080     EP-A1- 2 289 484
WO-A1-2007/095324     WO-A2-2009/086260
WO-A2-2011/048570

Remarks:
The file contains technical information submitted after the application was filed and not included in this specification

**Description**

Field of the Inventions

The invention relates to modified particle-polymer hybrids for use in skin care formulations. More specifically, the invention relates to a sun care formulation comprising a polymerizable monomer grafted onto a functionalized particle.

Background of the Invention

In certain skin care applications, such as sunscreens or facial and body lotions, milks and creams, foundations and other color cosmetics, particles have been included to improve the ability of the product to reflect or scatter ultraviolet light, or are used to protect light or oxygen sensitive cosmetic ingredients. In many cases, however, these insoluble particles do not provide adequate water resistance and polymers have been added to confer such benefits. Unfortunately, the use of polymers imparts undesirable thickening and tack in the skin care formulations. Thus, there is a need for skin care formulations that provide improved performance characteristics with minimal undesirable sensory effects.

WO 2009//086260 discloses a powder treated with a (meth)acrylic-grafted silicone polymer, the treated powder being obtained from a mixture containing a volatile silicone oil, a (meth)acrylic-grafted silicone polymer, in which a monomer component comprising a (meth)acrylic series monomer has been grafted onto the main chain of a polysiloxane, and a powder. The powder can be contained in a cosmetic product WO 2007/095324 discloses personal care compositions comprising responsive particles comprising a particle core having an interfacial surface and at least one block copolymer.

Summary of the Invention

Accordingly, the present invention is directed to skin care formulations that provide improved water resistance with limited impact on the sensory feel of the formulations, (e.g., tack). In an aspect, the invention provides a skin care formulation comprising a dispersion or emulsion comprising at least one particle-polymer hybrid and a cosmetically acceptable additive. The polymer-particle hybrid comprises at least one particle grafted with at least one polymer.

Detailed Description of the Invention

The present invention relates to skin care formulations and the preparation of skin care formulations that provide improved water resistance with limited impact on the sensory feel of the formulations (e.g. tack) due to their unique architecture. Generally, the invention is directed to skin care formulations comprising a dispersion or emulsion comprising at least one particle-polymer hybrid wherein the polymer-particle hybrid is formed by functionalizing at least one hydroxyl-containing particle with at least one linking group prior to grafting and grafting at least one polymerizable monomer that is capable of being grafted using free radical polymerization to the functionalized particle; , and a cosmetically acceptable additive. Such grafted particles which have at least one polymer grafted thereon provide materials that have unique properties useful in skin care formulation. The grafted particle-polymer hybrids are formed as dispersions, such as a lacquer, and/or emulsions that can ultimately be converted to dispersions or emulsions that can be incorporated into skin care formulations. When applied to the skin, the skin care formulation dries and forms a protective film on the surface of the skin.

In an aspect, the invention provides a skin care formulation comprising at least at least one particle-polymer hybrid wherein the hybrid comprises at least one particle grafted with at least one polymer, and a cosmetically acceptable additive. The particles are hydroxyl-containing particles that are at least partially functionalized with at least one linking group that is capable of participating in a free radical polymerization process, for instance, through initiation, propagation, fragmentation or transfer. In an embodiment of the invention, the linking groups can include, but are not limited to vinyl, thiol, or disulfide groups or combinations thereof. In further embodiments, mixed functionalization of particles is possible as well as particles bearing combinations of functionalities.

The functionalized hydroxyl containing particles are then polymerized with at least one monomer using conventional polymerization techniques that are known to those of ordinary skill in the art. In an embodiment of the invention, free radical polymerization is conducted using solution polymerization. Without wishing to be bound by theory, it is believed that the grafted polymer-particle hybrid has a large number of branches that allow for a high level of entanglement at high density due to interaction of a grafted particle with several other particles. The skin care formulations made from these materials, and the films that may be ultimately made therefrom, are dense, though they are not believed to be crosslinked. As entanglement and film formation thus do not involve very high molecular weight polymers, this results in limiting thickening and tack in skin care formulations, as well as improved integrity of the film.

In an embodiment of the invention, the particles included as the backbone or core for the grafting of the monomers may be made from a variety of materials. Suitable particle materials for use in the present invention can include, but are not limited to, organic materials or inorganic materials. Examples of inorganic materials may include metals, inorganic oxides, inorganic sulfides, inorganic antimonides, inorganic salts, inorganic nitrides, metallic particles, metal coated particles, clays, such as perkalite, hectorite, and the like. Metal particles can include particles of gold, platinum, silver, nickel and combinations of two or more thereof. Examples of organic materials include carbon black and organic pigments, for example, as well as other organic materials, such as polysaccharides. Examples of suitable polysaccharides include, but are not limited to, starch and its derivatives, cellulose and its derivatives, such as hydroxyalkylated and alkyl-substituted cellulose, carboxy-substituted polymers, xanthan, chitosan and guar.

Inorganic pigments may also be used such as red and yellow iron oxides, yellow 763ED (Pb chromate), green Co (Al, Cr)$_2$O$_4$, sodium alumino sulphosilicate (ultramarine blue), strontium carbonate, zinc phosphate, magnesium carbonate hydroxide and combinations of two or more of the foregoing. Inorganic oxides may be suitable for use in the process of the invention. Suitable oxides include zirconia, aluminum oxide, aluminum zirconium tetrachlorohydrex and its glycine adduct, calcium hydroxide, copper (II) hydroxide, lithium hydroxide, magnesium hydroxide, aluminum, aluminum hydroxide, titanium dioxide (titanium (IV) oxide), iron oxide, zinc oxide, silicon dioxide (silica), antimony trioxide, boron oxide, boron suboxide, bismuth (III) oxide, copper (I) oxide, copper (II) oxide, chromium (III) oxide, iron (II) oxide, iron (III) oxide, magnesium oxide - MgO, manganese (IV) oxide (manganese dioxide). Combinations of any two or more of the foregoing particulate materials are also included as embodiments of the invention.

In further embodiments, suitable particles may include particles of inorganic sulfides, including, but not limited to, copper (I) sulfide, copper (II) sulfide and zinc sulfide. In still further embodiments, suitable particles include particles of indium phosphide, aluminum phosphide, brass, metal coated glass, boron carbide, boron nitride, calcium carbide, aluminum sulfate, aluminosilicates such as mica, calcium sulfate, cobalt (II) carbonate, copper (II) carbonate, copper (II) nitrate, copper (II) sulfate, lithium carbonate, lithium nitrate, lithium sulfate, magnesium carbonate, magnesium phosphate, magnesium sulfate, manganese (II) sulfate monohydrate, manganese (II) phosphate, nickel (II) carbonate, nickel (II) hydroxide, nickel (II) nitrate, , zinc carbonate, zinc sulfate. Combinations of any two or more of the foregoing particulate materials are also included as embodiments of the invention.

In an embodiment of the invention, the particles are silicon dioxide (SiO2 or "silica"). Suitable silicon dioxide particles can be provided as nanoparticles or as microparticles. In some embodiments, silicon dioxide particles are solid particles (e.g., not hollow). In certain embodiments, silicon dioxide particles can be provided as hollow glass microspheres. In other embodiments, silicon dioxide particles can be provided as solid (e.g., not hollow) glass beads. In some embodiments, colloidal silica is preferred. Other colloidal metal oxides can be utilized such as colloidal titania, colloidal alumina, colloidal zirconia, colloidal vanadia, colloidal chromia, colloidal iron oxide, colloidal tin oxide, and mixtures of two or more of the foregoing. In an embodiment, colloidal particles can comprise a single oxide such as silica or can comprise a core of an oxide of one type over which is deposited an oxide of another type. In some embodiments, a suitable colloidal particle comprises a core of a material other than a metal oxide over which is deposited a metal oxide. In other embodiments, the particles can be used to encapsulate cosmetically acceptable ingredients, such as sun-protection agents, as are available from Aquea Scientific as Aquea SPFx.

In an embodiment of the invention, the particles include SiO$_2$, ZrO$_2$, TiO$_2$, CeO$_2$, ZnO, and combinations thereof, as well as three-dimensionally crosslinked organosilsesquioxane structures and metal oxides/hydroxides, in particular silsesquioxane structures (for example, known under the trade name POSS™ from Hybrid Plastics), or heteropolysiloxanes, particularly cubic or other three-dimensional representatives of this class of materials. Hybrids of nanoparticles and silsesquioxane structures can likewise be employed. Furthermore, particles based on phyllosilicates, sulfates, silicates, carbonates, nitrides, phosphates and sulfides of corresponding size may also be suitable. A further suitable particle material comprises particles selected from organic polymers/oligomers, in particular organic nanoparticles, for example consisting of free-radical-polymerized monomers. Dendrimers or hyperbranched polymers may also be used.

In another embodiment, the particles can include polymeric microspheres comprising about 1 micron functionalizable acrylate microspheres and spheres comprising a polymer-coated material, such as magnetic microspheres known as BIOMAG® dextran-coated charcoal, which is available from Polysciences, Inc. In another embodiment, the particles may be core-shell particles, hollow particles, liquid filled particles, fibrous particles, such as micronized cellulose, colloidal dispersions of inorganic particles (sols), such as described in WO 2009/131910.

Certain particles, such as silicas, can be obtained via a variety of processes, for example, by precipitation, flame pyrolysis, and grinding. Such processes for producing such particles are known to those of ordinary skill in the art. The processes can give a variety of structures, from primary particles to aggregates to agglomerates. The scope of the invention encompasses these various structures.

In an embodiment of the invention the particles are nanoparticles having an average particle size in the range of from about 10 nm to about 100 nm. In another embodiment, the nanoparticles have a size in the range of from about 10 nm to 20 nm to about 100 nm to 200 nm, and in another embodiment, of from about 200 nm up to about 1 micron. It is to be understood that for embodiments including ranges as described herein, the respective lower endpoints and respective upper endpoints described include combinations of the various lower and upper endpoints.

In another embodiment, the particles are microparticles having an average particle size in the range of from greater than about 1 micron up to about 500 microns. In another embodiment, the microparticles have a size in the range of from about 10 microns to about 500 microns, and in another embodiment, of from about 100 to about 500 microns. In still yet another embodiment, the microparticles have a size in the range of from greater than about 1 micron to about 100 microns and in another embodiment from greater than about 1 micron up to about 10 microns, and in yet another embodiment from of about 10 to about 100 microns.

In embodiments of the invention, the particles are hydroxyl containing particles that have been at least partially functionalized with linkers, such as organofunctional silanes. In an embodiment of the invention the functionalized silanes contain an ω-moiety capable of undergoing an initiation, transfer or propagation reaction in a free radical polymerization process. In an embodiment of the invention, the linker is at least one organofunctional silane. Suitable organofunctional silanes include, but are not limited to, vinyl- or thiol- functional silanes or non-terminal disulfide groups, such as bis(3-triethoxysilylpropyl)disulfide and bis(3-triethyoxysilylpropyl)tetrasulfide, and combinations thereof. In an embodiment, the functionalized particles may be obtained by reaction, such as by condensation, of a mercaptopropyltrialkoxysilane on the particle. In another embodiment of the invention, the organosilane is a methacryloyl group as obtained when condensing with a methacryloyloxyalkylenetrialkoxysilane. In another embodiment, the functionalization of particles may be obtained by condensation with a silane reagent bearing multiple functionalities, for example hydrophobic groups, such as VP Si 363 and the like available from Degussa. Other useful linkers are allyl-functionalized trialkoxysilane and vinyldimethyl functionalized trialkoxysilanes. Such functionalizing agents are described in WO 2009/131910. Other surface treatments include, without limitation, organotitanates and organothiols.

In a further embodiment, grafting of particles with polymers involves the functionalization of particles with functional entities associated with non traditional commercial polymerization processes including reversible addition/fragmentation chain transfer ("RAFT") polymerization , atom transfer radical polymerization ("ATRP"), and nitroxide-mediated polymerization ("NMP"). Other means to incorporate vinyl moieties into polymeric structures include cationic and anionic polymerization.

In another embodiment, such as for clays, attachment of a functional moiety can occur through an ionic bonding on the surface of the particle with a thiol- or vinyl-bound quaternary ammonium group. Examples of such linkers include ammonium groups bearing alike or non-alike substituents as in 3-(trimethoxysilyl)propyldimethyloctodecylammonium chloride, further condensed with a thiol or vinyl-bound silane.

In yet another embodiment, grafting of the hydroxyl functionalized particle using an alkoxysilane functional approach allows for the functionalization of the particle in a single step that makes it directly useful to graft polymers, and may involve traditional free radical polymerization techniques.

In an embodiment, during the grafting operation, the modified (functionalized) particle may be used in levels of from about 0.1% to about 50% by weight of the preparation of the hybrid polymer particle. In another embodiment, the modified particle may be present in an amount of from about 10% to about 30% by weight, and in another embodiment of from about 10% to about 25%.

In accordance with the present invention, the functionalized particles as the core are grafted with polymerizable monomers. The polymerizable monomers are monomers capable of being grafted using free radical polymerization. Suitable monomer classes include acrylates, methacrylates, acrylamides and methacrylamides, styrene and its derivatives, vinyl esters and ethers, vinyl amides, vinylpyrrolidone and vinyl caprolactam. A more complete listing is described in US 2010/0278764. In an embodiment of the invention, the polymerizable monomers are chosen from methyl acrylate, ethyl acrylate, propyl acrylate, butyl acrylate, t-butyl acrylate, ethylhexyl acrylate, behenyl acrylate, methyl methacrylate, ethyl methacrylate, propyl methacrylate, hydroxyethyl acrylate and methacrylate, hydroxypropyl acrylate and methacrylate, cyclohexyl acrylate, dimethylaminoethyl methacrylate, dimethylamidopropyl methacrylamide, methacrylic acid, acrylic acid, benzyl acrylate, 2-Acrylamido-2-methylpropane sulfonic acid and its salts, vinyl acetate, vinyl propionate, vinyl neodecanoate,diallyl dimethyl ammonium chloride, dimethylaminoethyl methacrylate, dimethylaminoethyl methacrylate diethyl sulfate, trimethylaminoethyl methacrylate chloride, N,N-Dimethylaminoethyl methacrylamide and its quaternary ammonium derivatives, vinyl pyrrolidone, vinyl caprolactam, vinyl formamide, sodium styrene sulfonate, itaconic and maleic acids and derivatives, vinyl ethers, and polymerizable surfactants, and combinations thereof.

The functionalized particles are polymerized with at least one monomer and, for example in the case of solution polymerization, the polymerization is in the presence of a solvent or diluent. In an embodiment of the invention, suitable solvents or diluents include organic solvents, such as methyl ethyl ketone, tetrahydrofuran, lower boiling alcohols, such as methanol, ethanol, propanol, propanediol and butanol, and esters, such as ethyl acetate and isopropyl acetate, carbonates, including cosmetically acceptable oils and cosmetically acceptable solvents, such as phenethyl alcohol, and emollients, such as caprylyl caprylate. A description of these and cosmetically acceptable oils are described in US 2010/0278764. In the case of emulsion or suspension polymerization, the diluent is water, and the polymerization takes place in the dispersed phase which may or may not contain organic solvents such as those listed above. In particular, it is conceivable that the functionalized particles in an organic solvent are dispersed in an aqueous environment prior to polymerization. It is also possible to convert the grafted polymer-particle containing lacquer to an aqueous system by exchanging the diluent, if such is preferred in the formulation of the hybrid material.

In another embodiment, the particles are functionalized with the silane reagent in the presence of all or part of the monomers used to create the hybrid, and polymerized as a suspension or emulsion process with water as the diluent.

In an embodiment of the invention, the particle-polymer hybrids are present in amounts of from about 1% to about 25% of the skin care formulations. In another embodiment, the particle-polymer hybrids are present in amounts of from about 5% to about 20% of the skin care formulation, and in even further embodiments, of from about 10% to about 15% of the skin care formulations.

In addition to the particle-polymer hybrid, the skin care formulations further include cosmetically acceptable additives. In an embodiment of the invention, the polymer-particle hybrids are mixed with a cosmetically acceptable additive to provide a skin care

formulation. The polymer-particle hybrids may be added as an aqueous system to the cosmetically acceptable additive, or they may be added as a dispersion or an emulsion to the skin care formulation. Such cosmetically acceptable additives may include, but are not limited to the following actives, agents and optional ingredients.

For purposes of the present invention, a "sunscreen active agent" or "sunscreen active" shall include all of those materials, singly or in combination, that are regarded as acceptable for use as active sunscreening ingredients based on their ability to absorb UV radiation. Such compounds are generally described as being UV-A, UV-B, or UV-A/UV-B active agents. Approval by a regulatory agency is generally required for inclusion of active agents in formulations intended for human use. Those active agents which have been or are currently approved for sunscreen use in the United States include organic and inorganic substances including, without limitation, para aminobenzoic acid, avobenzone, cinoxate, dioxybenzone, homosalate, menthyl anthranilate, octyl salicylate, oxybenzone, padimate O, phenylbenzimidazole sulfonic acid, sulisobenzone, trolamine salicylate, titanium dioxide, zinc oxide, diethanolamine methoxycinnamate, digalloy trioleate, ethyl dihydroxypropyl PABA, glyceryl aminobenzoate, lawsone with dihydroxyacetone, red petrolatum. Examples of additional sunscreen actives that have not yet been approved in the US but are allowed in formulations sold outside of the US include ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidenecamphor, and isopentyl 4-methoxycinnamate. However, as the list of approved sunscreens is currently expanding, those of ordinary skill will recognize that the invention is not limited to sunscreen active agents currently approved for human use, but is readily applicable to those that may be allowed in the future.

In one embodiment of the invention the sunscreen active agent comprises a photoprotecting effective amount of particulates of at least one inorganic pigment or nanopigment that is present in addition to the particle polymer hybrid and might be based on the therein particle species. Non-limiting examples of which include titanium dioxide, zinc oxide, iron oxide, silicon dioxide, zirconium oxide, cerium oxide, or mixture thereof.

The compositions of this invention can be applied to the skin as a liquid rub on. However, the compositions are not limited to those compositions applied to the skin primarily as a sunscreen agent. The skin care formulations of the invention may include compositions that also incorporate those formulations where the sunscreen active agent may be included as an ingredient in another topically applied composition. Some non-limiting examples are lipstick, make-up, lip-balm, eye-shadow, eye-liner, foundations or any application, and in particular where sun protection may be deemed beneficial.

The compositions of the present invention may contain a wide range of additional, optional components which can also include components generally known as botanical agents. The CTFA Cosmetic Ingredient Handbook, Seventh Edition, 1997 and the Ninth Edition, 2002, particularly at page XV, describes a wide variety of cosmetic and botanical agents and active ingredients commonly used in skin care compositions, which are suitable for use in the compositions of the present invention. Examples of these functional classes disclosed in this reference include: absorbents, abrasives, anticaking agents, antifoaming agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, plasticizers, reducing agents, skin bleaching agents, skin-conditioning agents (emollients, such as silicones, humectants, miscellaneous, and occlusive), skin protectants, solvents, foam boosters, hydrotropes, solubilizing and emulsifying agents, suspending agents (nonsurfactant), sunscreen agents, ultraviolet light absorbers, SPF boosters, waterproofing agents, and viscosity increasing agents (aqueous and nonaqueous).

The formulations of the invention may also include materials that also increase the SPF of the final composition by such mechanisms as UV radiation scattering and dispersion. Such materials are referred to herein as "UV-radiation scattering agents" and comprise materials that exhibit UV absorbing activity or exhibit no UV absorbing activity. An example of such UV-radiation scattering agents include polymeric materials, such as the product known as SunSpheres® (Rohm and Haas; Philadelphia, Pa.) which are described by their manufacturer as hollow styrene/acrylates copolymer spheres manufactured by emulsion polymerization. The polymer spheres are said to raise SPF values across the UVA and UVB region by dispersing and/or scattering the incident UV radiation throughout the film of sunscreen present on a surface, such as human skin. It is understood that the spheres cause less UV radiation to penetrate into the skin by redirecting the radiation towards the UV-absorbing sunscreen actives in the sunscreen formulation, where the radiation reacts with the sunscreen active molecules and the energy is dissipated as heat. As used herein, the terms "spheres" or "scattering agents" are not limited by chemical makeup or shape, but comprise any agent that produces the effect of lengthening the path of incident UV radiation, increasing the statistical likelihood that the radiation will contact a sunscreen active molecule, i.e., a UV absorbing active agent. These materials may also include UV absorbing materials that also exhibit scattering properties such as ZnO (examples include Z-Cote® products available from BASF), TiO$_2$ (examples include the Solaveil® products available from Croda (New Castle, Del., USA)), compounds such as methylene bis-benzotriazolyl tetramethylbutylphenol, (Tinasorb® M available from Ciba Specialty Chemicals, Inc. (Basel, Switzerland). UV radiation scattering agents are typically present in the formulation in amounts up to about 25% by weight. Certain example embodiments of the invention may comprise up to about 10% by weight, preferably in ranges of about 0.5% to about 7.0% by weight, in particularly preferred ranges of 3% to about 5% by weight.

As used herein, the terms "sunless-tanning agent" or "self-tanning compositions" refer to compositions which, when applied to human skin, impart thereto an appearance similar to that achieved by exposing the skin to natural or artificial sunlight. Examples of sunless tanning active agents are described in U.S. Pat. Nos. 6,482,397, 6,261,541, and 6,231,837. Such sunless tanning compositions typically comprise, in addition to an artificial tanning effective amount of a self tanning agent, effective amounts of a composition coloring agent and a cosmetically acceptable carrier adapted for topical application to human skin. The self tanning agents can also include those compositions generally accepted in the art for application to human skin, and which, when so applied, react therein with amino acids so as to form pigmented products. Such reactions give the skin a brown appearance similar to the color obtained upon exposing it to sunlight for periods of time sufficient to tan the skin. Suitable self tanning agents include, without limitation, alpha-hydroxy aldehydes and ketones, glyceraldehyde and related alcohol aldehydes, various indoles, imidazoles and derivatives thereof, and various approved pigmentation agents. Presently preferred herein as self tanning agents are the alpha-hydroxy aldehydes and ketones. Most preferably, the self tanning agent is dihydroxyacetone ("DHA").

Other suitable self tanning agents include, without limitation, methyl glyoxal, glycerol aldehyde, erythrulose, alloxan, 2,3-dihydroxysuccindialdehyde, 2,3-dimethoxysuccindialdehyde, 2-amino-3-hydroxy-succindialdehyde and 2-benzylamino-3-hydroxysuccindialdehyde.

An emollient is an oleaginous or oily substance which helps to smooth and soften the skin, and may also reduce its roughness, cracking or irritation. Typical suitable emollients include mineral oil having a viscosity in the range of 50 to 500 centipoise (cps), lanolin oil, coconut oil, cocoa butter, olive oil, almond oil, macadamia nut oil, aloe extracts such as aloe vera lipoquinone, synthetic jojoba oils, natural sonora jojoba oils, safflower oil, corn oil, liquid lanolin, cottonseed oil, grape seed oil, sweet almond oil, and peanut oil. Preferably, the emollient is an alkyl benzoate sold under the trade name Finsolv TN from Finetex. One or more emollients may be present ranging in amounts from about 1 percent to about 10 percent by weight, preferably about 5 percent by weight. Another suitable emollient is DC 200 Fluid 350, a silicone fluid, available Dow Corning Corp.

Other suitable emollients include squalane, castor oil, polybutene, sweet almond oil, avocado oil, calophyllum oil, ricin oil, vitamin E acetate, olive oil, silicone oils such as dimethylopolysiloxane and cyclomethicone, linolenic alcohol, oleyl alcohol, the oil of cereal germs such as the oil of wheat germ, isopropyl palmitate, octyl palmitate, isopropyl myristate, hexadecyl stearate, butyl stearate, decyl oleate, acetyl glycerides, the octanoates and benzoates of (C.sub.12-C.sub.15) alcohols, the octanoates and decanoates of alcohols and polyalcohols such as those of glycol and glyceryl, ricinoleates esters such as isopropyl adipate, hexyl laurate and octyl dodecanoate, dicaprylyl maleate, hydrogenated vegetable oil, phenyltrimethicone, jojoba oil and aloe vera extract.

Other suitable emollients which are solids or semi-solids at ambient temperatures may be used. Such solid or semi-solid cosmetic emollients include glyceryl dilaurate, hydrogenated lanolin, hydroxylated lanolin, acetylated lanolin, petrolatum, isopropyl lanolate, butyl myristate, cetyl myristate, myristyl myristate, myristyl lactate, cetyl alcohol, isostearyl alcohol and isocetyl lanolate. One or more emollients can optionally be included in the formulation.

A humectant is a moistening agent that promotes retention of water due to its hygroscopic properties. Suitable humectants include glycerin, polymeric glycols such as polyethylene glycol and polypropylene glycol, mannitol, hydroxyalkyl urea and sorbitol. One or more humectants can optionally be included in the formulation in amounts from about 1 percent to about 10 percent by weight, preferably about 5 percent by weight.

A dry-feel modifier is an agent which when added to an emulsion, imparts a "dry feel" to the skin when the emulsion dries. Dry feel modifiers can include talc, kaolin, chalk, zinc oxide, silicone fluids, inorganic salts such as barium sulfate, surface treated silica, precipitated silica, fumed silica such as an Aerosil available from Degussa Inc. of New York, N.Y. U.S.A. Another dry feel modifier is an epichlorohydrin cross-linked glyceryl starch of the type that is disclosed in U.S. Pat. No. 6,488,916.

It may be advantageous to incorporate additional thickening agents, such as, for instance, various Carbopols available from Noveon Co. Particularly preferred are those agents which would not disrupt the lamellar structure in the formulation of the final product, such as non-ionic thickening agents. The selection of additional thickening agents is well within the skill of one in the art.

Additional natural or synthetic substances can also be added to the compositions of the invention to protect from or delay its deterioration due to the action of oxygen in the air (oxidation). They may also reduce oxidation reactions in skin tissue. Such substances prevent oxidative deterioration which may lead to the generation of rancidity and non-enzymatic browning reaction products. Typical suitable substances include propyl, octyl and dodecyl esters of gallic acid, butylated hydroxyanisole (BHA, usually purchased as a mixture of ortho and meta isomers), butylated hydroxytoluene (BHT), green tea extract, uric acid, cysteine, pyruvate, nordihydroguaiaretic acid, Vitamin A, Vitamin E and Vitamin C and their derivatives. One or more such substances can optionally be included in the composition in an amount ranging from about 0.001 to about 5 weight percent, preferably about 0.01 to about 0.5 percent.

Chelating agents are substances used to chelate or bind metallic ions, such as with a heterocylic ring structure so that the ion is held by chemical bonds from each of the participating rings. Suitable chelating agents include ethylene diaminetetraacetic acid (EDTA), EDTA disodium, calcium disodium edetate, EDTA trisodium, albumin, transferrin, desferoxamine, desferal, desferoxamine mesylate, EDTA tetrasodium and EDTA dipotassium, glutamic acid diacetic acid salts or combinations of any of these.

Fragrances are aromatic substances which can impart an aesthetically pleasing aroma to the sunscreen composition. Typical fragrances include aromatic materials extracted from botanical sources (i.e., rose petals, gardenia blossoms, jasmine flowers, etc.) which can be used alone or in any combination to create essential oils. Alternatively, alcoholic extracts may be prepared for compounding fragrances. However, due to the relatively high costs of obtaining fragrances from natural substances, the modern trend is to use synthetically prepared fragrances, particularly in high-volume products. One or more fragrances can optionally be included in the sunscreen composition in an amount ranging from about 0.001 to about 5 weight percent, preferably about 0.01 to about 0.5 percent by weight.

Additional preservatives may also be used if desired and include well known preservative compositions such as benzyl alcohol, phenyl ethyl alcohol and benzoic acid, diazolydinyl, urea, chlorphenesin, iodopropynyl butyl carbamate, and ethylhexyl glycerin among others.

The compositions of the invention can further comprise skin protectant active agents. Suitable examples include (with preferred weight percent ranges), Allantoin (0.5 to 2 percent); Aluminum hydroxide gel (0.15 to 5 percent); Calamine (1 to 25 percent); Cocoa butter (greater than 50); Cod liver oil (5 to 14 percent); Colloidal oatmeal; Dimethicone (1 to 30 percent); Glycerin (20 to 45 percent); Hard fat (greater than 50); Kaolin (4 to 20 percent); Lanolin (12.5 to 50 percent); Mineral oil (greater than 50 percent); Petrolatum (greater than 30 percent); Sodium bicarbonate; Topical starch (10 to 98 percent); White petrolatum (greater than 30 percent); Zinc acetate (0.1 to 2 percent); Zinc carbonate (0.2 to 2 percent).

The compositions of the invention may further include insect repelling components. The most widely used insect repelling active agent for personal care products is N,N-Diethyl-m-toluamide, frequently called "DEET" and available in the form of a concentrate containing at least about 95 percent DEET. Other synthetic chemical repellents include ethyl butylacetylaminoproprionate (also known as IR 3535), dimethyl phthalate, ethyl hexanediol, indalone, di-n-propylisocinchoronate, bicycloheptene, dicarboximide and tetrahydrofuraldehyde. Certain plant-derived materials also have insect repellent activity, including citronella oil and other sources of citronella (including lemon grass oil), limonene, rosemary oil and eucalyptus oil. Choice of an insect repellent for incorporation into the sunscreen emulsion will frequently be influenced by the odor of the repellent. The amount of repellent agent used will depend upon the choice of agent; DEET is useful at high concentrations, such as up to about 15 percent or more, while some of the plant-derived substances are typically used in much lower amounts, such as 0.1 percent or less.

The compositions of the present invention may also optionally include at least one stabilizing polymer to impart rheological stability to the compositions. Suitable stabilizing polymers can include, but are not limited to, acrylic acid-based systems such as Carbomer® 940 and 980 available from The Lubrizol Corporation, Pemulen® polymers available from The Lubrizol Corporation, xanthan gums, such as Amaze® XT available from Akzo Nobel Surface Chemistry LLC, cellulosic thickeners like the Structure®-Cel products available from Akzo Nobel Surface Chemistry LLC, and starch thickeners, such as Structure® XL available from Akzo Nobel Surface Chemistry LLC, and combinations thereof.

The compositions of the present invention may further optionally include at least one waterproofing polymer. Suitable waterproofing polymers include, , but are not limited to, VP/eicosene, such as Ganex® V-220 available from International Specialty Products, acrylates copolymers, such as Dermacryl® AQF available from Akzo Nobel Surface Chemistry LLC, sulfopolyesters, such as Eastman AQ™ 38S available from Eastman Chemical, polyesters, such as LexFilm® Sun available from Inolex Chemical Company, and SPF boosters, such as Syntran® PC 5227 available from Interpolymer Corporation, and combinations thereof.

In still yet another embodiment, the skin care formulations of the present invention may be sprayable formulations, such as sprayable sunscreen formulations. Accordingly, in an embodiment of the invention ,the sprayable skin care formulations may comprise from about 38 to 92% of a carrier, such as ethanol or dimethyl ether, up to 25% by weight of one or more sunscreen actives and from about 0.1 to about 5% of a film former. In a further embodiment of the sprayable skin care formulations, dimethicone derivatives may be included to increase the water resistance, as described in US 2009/0035234.

In another aspect, the invention relates to a method for preparing the skin care formulation comprising polymerizing at least one partially functionalized particle with at least one monomer in the presence of a solvent or diluent; mixing the resulting grafted particle-polymer hybrid with a cosmetically acceptable additive; and optionally converting the grafted polymer-particle hybrid to an aqueous system. The method comprises reacting a micro- or nano- hydroxyl containing particle with a linker, such as a vinyl- or a thiol- functional silane to form a functionalized particle. In an embodiment of the invention, the thiol-functional particles may be obtained by condensation of mercaptopropyltrialkoxysilane on the particle. The method further comprises polymerizing the functionalized particle with at least one monomer, preferably in the presence of an organic solvent.

The product of the polymerization reaction is a particle-polymer hybrid wherein the particle has at least one monomer grafted to it. In a further step, the organic solvent is removed after polymerization and the solvent replaced with an aqueous carrier. The aqueous carrier may incorporate sodium hydroxide and/or a surfactant. In an embodiment of the invention, the surfactant may be sodium laureth sulfate. Optionally, treatment with hydrogen peroxide may be used to reduce any remaining thiol groups.

In an embodiment of the invention, the skin care formulation has an intrinsic viscosity, IV, in the range of from about 0.1 to about 2. In another embodiment, the intrinsic viscosity, IV, is in the range of from about 0.5 to about 1.5. In yet another embodiment, the intrinsic viscosity, IV, is in the range of from about 0.75 to about 1.0. If the intrinsic viscosity is too high, then the formulation will be too difficult to process. On the other hand, if the intrinsic viscosity is too low, the skin care formulation may not provide sufficient water resistance or integrity to the formulation.

The grafted particle-polymer hybrids provide better uniformity of the actives, improved dispersability, and less tack in the sun care formulations.

Examples

The following examples are intended to exemplify the present invention but are not intended to limit the scope of the invention in any way. The breadth and scope of the invention are to be limited solely by the claims appended hereto.

Water Resistance Screening Test of Formulations

In this test, 0.2 g of the formulation are applied to a wool swatch in a circle measuring 1.5" in diameter, spreading the sample in 15 s using a finger cot. The swatch is then allowed to dry in a controlled environment (50% relative humidity, 21C) for 15 min. The dried swatch is attached, using a binder clip, to the wall of the 150 ml beaker, having the treated side of the swatch facing inwards and dipped into the water. We then add 145 mls of warm water (36°-37°C) and stir bar to the beaker, and cover the mouth of the beaker with aluminum foil. The water is then stirred for 15 min.

**Subjective test:** Visual inspection of aqueous phase

Cloudy = low water resistance (sunscreen washing off swatch).
Clear = indication that the film forming polymer in the sunscreen formulation holds it onto the swatch.

**Objective test:** Turbidity Test

Low reading (relatively clear water) = majority of sunscreen remains on swatch
High reading (cloudy water) = sunscreen washing off into water

Preparation of the particle-polymer hybrid precursor

In each of the following examples, the particle-polymer hybrid precursor was made according to the following procedure. A round-bottom flask was equipped with mechanical agitation and a condenser. A mixture of about 10% of the various monomers and the thiol-functional particles (obtained by condensation of mercaptopropyltrimethoxysilane on the particle) were added, along with the organic solvent for the polymerization. The monomers were polymerized in the presence of the functional particles, with slow-addition of the remainder of the reagents. The particle-polymer hybrid precursor can be recovered by replacing the organic medium for the polymerization with an aqueous carrier, which incorporated sodium hydroxide or sodium and a surfactant, such as sodium laureth sulfate.

Determination of intrinsic viscosity (IV) of the particle-polymer hybrid precursor

A relative comparison of the MW of the polymer particle hybrids was performed by measuring the IV of the polymer particle lacquers at ambient temperature. The IV (intrinsic viscosity) is calculated by the formula

$$IV = \frac{3}{c} (\eta_{rel}^{1/3} - 1)$$ in which c is the concentration (g/100 ml of solution, which is typically around 0.8g/100ml), and $\eta_{rel}$ is the ratio of the flow time of the solution of the polymer divided by the flow time for the solvent in the capillary tube.

Preparation of "Organic' Sunscreen Formula

A base formulation and procedure for preparing an "organic sunscreen" follows. In the control, DERMACRYL® AQF acrylates copolymer (sold by AkzoNobel) was included at a concentration of 2.0% on a dry basis of the formulation. For the examples according to embodiments of the present invention, the particle-polymer hybrid was substituted for the DERMACRYL® AQF acrylates copolymer in the formulation. The concentration of the particle-polymer hybrid was the same as that for the DERMACRYL® AQF acrylates copolymer in the formulation.

| "Organic" Sunscreen Formulatoin | | |
|---|---|---|
| | | |
| INCI Name | Trade Name | % |
| | | |
| Phase A | | |
| Isohexadecane | Arlamol HD | 1.50 |
| C12-15 Alkyl Benzoate | Finsolv TN | 3.00 |
| Cyclopentasiloxane | DC Fluid 245 | 2.25 |
| Sorbitan Stearate | Protachem SMS | 1.00 |
| Glyceryl Stearate (and) PEG 100 Stearate | Arlacel 165 | 2.00 |
| Octocrylene | Neo Heliopan 303 | 2.00 |
| Ethylhexyl Methoxycinnamate | Neo Heliopan AV | 7.50 |
| Benzophenone - 3 | Neo Heliopan BB | 3.00 |
| Zinc Oxide (and) C12-15 Alkyl Benzoate (and) | Spectraveil FIN | 6.00 |
| Polyhydroxystearic Acid | | |
| | | |
| Phase B | | |
| Water | Water | q.s. |
| Dehydroxanthan Gum | AMAZE XT Polymer | 0.50 |
| Acrylates Copolymer (45% act) or experimental polymer | | 2% dry basis |
| Glycerin | Glycerin | 3.00 |
| Titanium Dioxide (and) Alumina (and) Silica | Tioveil AQ-N | 7.00 |
| (and) Sodium Polyacrylate | | |
| | | |
| Phase C | | |
| DMDM Hydantoin (and) Idoipropynyl Burylcarbamate | Glydant plus liq. | 0.60 |

(continued)

| Phase C | | |
|---|---|---|
| Corn starch modified | DRY FLO AF PURE starch | 2.00 |
| Citric Acid | 50% Citric Acid soln | pH 7-7.5 |
| | total | 100 |
| | | |

Procedure:

1. Combine all of the ingredients in Phase A and begin heating to 75°C.
2. Disperse the AMAZE XT polymer into water until completely hydrated
3. Combine the rest of the ingredients in phase B to the hydrated AMAZE XT solution (EXCEPT TIOVEIL AQ-N).
4. Begin heating to 75°C.
5. Homogenize the Tioveil AQ-N and add this to Phase B at 75°C.
6. Add phase A to Phase B at 75C.
7. Homogenize at 10,000 rpm for 1 minute. Cool to 40°C with moderate stirring.
8. When formula reaches 40°C add phase C, one ingredient at a time until homogenous

Preparation of "Inorganic" Sunscreen Formula

A base formulation and procedure for preparing an "inorganic sunscreen" follows. In the control, DERMACRYL® AQF acrylates copolymer was included at a concentration of 2.0% on a dry basis of the formulation. For the examples according to embodiments of the present invention, the particle-polymer hybrid was substituted for the DERMACRYL® AQF acrylates copolymer in the formulation. The concentration of the particle-polymer hybrid was the same as that for the DERMACRYL® AQF acrylates copolymer in the formulation.

| "Inorganic" Sunscreen Formulatoin | | |
|---|---|---|
| INCI Name | Trade Name | % |
| | | |
| Phase A | | |
| Isohexadecane | Arlamol HD | 1.50 |
| C12-15 Alkyl Benzoate | Finsolv TN | 3.00 |
| Cyclopentasiloxane | DC Fluid 245 | 2.25 |
| Sorbitan Stearate | Protachem SMS | 1.00 |
| Glyceryl Stearate (and) PEG 100 Stearate | Arlacel 165 | 2.00 |
| Zinc Oxide (and) Polyhydroxystearic Acid | Spectraveil FIN | 20.00 |
| | | |
| Phase B | | |
| Water | Water | q.s. |
| Dehydroxanthan Gum | AMAZE XT Polymer | 0.50 |
| Acrylates Copolymer (45% act) or experimental sample | | 2% on dry basis |
| Glycerin | Glycerin | 3.00 |
| Titanium Dioxide (and) Alumina (and) Silica | Tioveil AQ-N | 23.00 |
| (and) Sodium Polyacrylate | | |

(continued)

| Phase B | | |
|---|---|---|
| | | |
| Phase C | | |
| DMDM Hydantoin (and) Idoipropynyl Butylcarbamate | Glydant plus liq. | 0.60 |
| Citric Acid | 50% Citric Acid soln | pH 7-7.5 |

Procedure:

1. Combine all of the ingredients in Phase A and begin heating to 75C.
2. Disperse the AMAZE XT polymer into water until completely hydrated.
3. Combine the rest of the ingredients in phase B to the hydrated AMAZE XT solution (EXCEPT TIOVEIL AQ-N).
4. Begin heating to 75C.
5. Homogenize the Tioveil AQ-N and add this to Phase be at 75C.
6. Add phase A to Phase B at 75C.
7. Homogenize at 10,000 rpm for 1 minute. Cool to 40C with moderate stirring.
8. When formula reaches 40C add phase C, one at a time until homogenous
9. Adjust pH to specification

Sunscreen Evaluation

Formulations were tested for viscosity, pH, and visual stability over a period of 4 weeks at 40° C. The above screening test for water resistance was conducted against the control formulations. The ratio of the turbidity of the sample over that of the control is an indication of the respective water resistance character of the formulas.

Examples 1-12 - MMA/BA/MAA polymer nanosilica hybrids prepared from MEK

Examples 1-12, as shown in Table 1, illustrate the experimental conditions and water resistance results for polymers of the same composition based on the monomers methyl methacrylate (MMA), butyl acrylate (BA), and methacrylic acid (MAA), prepared in methyl ethyl ketone (MEK). The emulsification process was done either with NaOH alone (as a neutralizer for MAA) or in combination of NaOH and sodium laureth sulfate as the surfactant. The nanosilicas used in these experiments were 10 nm in diameter and were obtained from Nissan Chemical America (MEK-ST). The formulations were stable and show in most cases a superior water resistance score than the controls, especially as the IV of the intermediate lacquer is increased. Example 12 in particular had a nice, smooth surface and feel.

Table 1

| Example | | 1 | 2 | 3 | 4 | 5 | 6 | 7 | 8 | 9 | 10 | 11 | 12* |
|---|---|---|---|---|---|---|---|---|---|---|---|---|---|
| SH/nanosilica, wt%# | | 9 | 4.51 | 4.51 | 4.51 | 4.51 | 4.51 | 4.51 | 4.51 | 4.51 | 4.51 | 4.51 | 4.51 |
| emulsification | | NaOH | NaOH/ surf | NaOH | NaOH | NaOH/ surf | NaOH | NaOH/ surf | NaOH/ surf | NaOH | NaOH/ surf | 50% more surf than in exp # 10 | NaOH |
| | | | | | | | | | | | | | |
| IV | | 0.33 | 0.27 | 0.27 | 0.35 | 0.35 | 0.35 | 0.35 | 0.37 | 0.41 | 0.41 | 0.41 | |
| | | | | | | | | | | | | | |
| organic formula ratio | WR/WR control | 0.55 | 0.52 | 0.72 | 0.72 | 0.31 | 0.58 | 0.38/0.62 | 0.65/0.37 | 0.35 | 0.43/0.68 | 0.76 | 0.64 |
| inorganic formula ratio | WR/WR control | 1.9 | 1.02 | 0.86 | 1.11/0.55 | 0.59 | 1.48 | 0.21 | 0.19/0.32 | 1.22 | 0.69 | 0.42 | 0.3 |

SH/nanosilica wt% = weight percent of the silane reagent to that of the particles
WR = water resistance screen test value
standard amount of surfactant was 1.3% on a dry basis over weight of polymer

EP 2 694 015 B1

Examples 13-20 - MMA/BA/MAA polymer - nanosilica hybrid controls

Examples 13-20, as shown in Table 2, show control experiments in which no nanosilica was used (exp 13 & 14), a non-thiol functionalized nanosilica was blended prior to the dispersion step in the polymer lacquer (exp 15 & 16), or the non-thiol functionalized nanosilica was present during the preparation of the polymer (experiments 19 & 20), against the use of a thiol-functional nanosilica during the preparation of the polymer (exp 17 & 18). The experiments with the thiol-functional particles (17 & 18) exhibit superior water resistance by the screening test than the control formulations, and overall outperform the systems where no nanosilica or a non-thiol functionalized nanosilica was used.

Table 2

| Example # | | 13 | 14 | 15 | 16 | 17 | 18 | 19 | 20 |
|---|---|---|---|---|---|---|---|---|---|
| SH/nanosilica, wt % | | **0** | **0** | **0** | **0** | **4.51** | **4.51** | **0** | **0** |
| conditions | | no nanosilica | no nanosilica | silica-OH prior to dispersion | silica-OH prior to dispersion | control with SH nanosilica | control with SH nanosilica | silica-OH during lacquer preparation | silica-OH during lacquer preparation |
| emulsification | | NaOH | NaOH / surfactant | NaOH | NaOH / surfactant | NaOH | NaOH / surfactant | NaOH | NaOH / surfactant |
| IV | | 0.29 | 0.29 | | | 0.41 | 0.41 | 0.34 | 0.34 |
| organic formula ratio | WR/WR control | 1.10 | 1.80 | 0.88 | 1.61 | 0.60 | 0.59 | 0.78 | 0.92 |
| inorganic formula ratio | WR/WR control | 1.00 | 0.62 | 0.98 | 0.59 | 0.89 | 0.89 | 1.57 | 1.22 |

Examples 21-22 - MMA/BA/MAA polymer with oblong nanosilica particle hybrids

Examples 21-22, as shown in Table 3, relate to the preparation of oblong nanosilica particle-based polymer hybrids from nanosilica particles having a length of about 40-100 nm and a diameter of about 9-15 nm. The monomers used in these preparations were also based on MMA, BA and MAA. The sunscreens based on these hybrids were stable and showed, in the case of the hybrids emulsified with the use of sodium hydroxide, superior water resistance that the control formulation using the Water Resistance Screening Test, as described hereinabove.

Table 3

| Example # | | 21 | 22 |
|---|---|---|---|
| **SH/nanosilica, wt %** | | **0.6** | **0.6** |
| emulsification | | NaOH | NaOH, surfactant |
| organic formula ratio | WR/WR control | 0.43 | 2.55 |
| inorganic formula ratio | WR/WR control | 0.61/0.63 | 1.59 |

Examples 23-25 - MMA/BA/MAA polymer nanosilica hybrids prepared from ethyl acetate

Examples 23-25, as shown in Table 4, illustrate the experimental conditions and water resistance results for polymers of the same composition based on the monomers MMA, BA, and MAA, prepared in ethyl acetate (EtOAc) with thiol-functionalized nanosilicas having a diameter of 10 nm. The emulsification process performed either with NaOH alone (as a neutralizer for MAA) or in combination of NaOH and sodium laureth sulfate. The results show a clear dependence of the water resistance screen test result on the means of isolation of the polymer-particle hybrid. The formulations were stable.

To reduce the viscosity of the inorganic sunscreen formulas, a modification was made to the formulation by reducing the level of dehydroxanthan gum by half. This is reported in experiments 26 and 27. The experiment where NaOH is used as a sole stabilizer, i.e. Example 26, shows a significant enhancement over the control, also prepared with a reduction in the gum loading. The reduction in the dehydroxanthan gum did not contribute to a loss of stability of the formulas.

Table 4

| Example # | | 23 | 24 | 25 | 26 | 27 |
|---|---|---|---|---|---|---|
| SH/nanosilica, wt % | | 4.51 | 4.51 | 4.51 | 4.51 | 4.51 |
| emulsification | | NaOH | NaOH/ surf (vs exp 23) | NaOH/50% more surf than in exp 23 | NaOH | NaOH/ surf (vs exp 23) |
| other modifications | | | | | as exp 23 with 0.25% dehydroxanthan gum | as exp 24 with 0.25% dehydroxanthan gum |
| IV | | 0.43 | 0.43 | 0.43 | 0.43 | 0.43 |
| organic formula ratio | WR/WR control | 0.69 | 0.94 | 0.65/0.70 | | |
| inorganic formula ratio | WR/WR control | 3.07 | 0.91/1.00 | 0.52/0.52 | 0.63 | 1.18 |

Examples 28-33 - MMA/BA/MAA polymer titanium dioxide hybrids

Examples 28-33, as shown in the Table 5, show the experimental conditions and water resistance results for polymers of the same composition based on the monomers MMA, BA, and MAA, prepared in MEK and using $TiO_2$. The emulsification process was done either with NaOH alone (as a neutralizer for MAA) or in combination of NaOH and sodium laureth sulfate. The titanium dioxide particles used in these experiments were en average 0.6 microns in diameter. The formulations were stable. Improvements in the water resistance screen test were observed with the organic sunscreen formulation, and compare favorably with polymers prepared without the presence of particles (Examples # 13 & 14)

Table 5

| Example # | | 28 | 29 | 30 | 31 | 32 | 33 | 13 | 14 |
|---|---|---|---|---|---|---|---|---|---|
| SH/particle, wt % | | 0.0013 | 0.013 | 0.06 | 0.06 | 0.06 | 0.06 | | |
| particle/monomer, wt% | | 7.68 | 15.37 | 7.69 | 15.38 | 7.69 | 15.38 | 0 | 0 |
| emulsification | | NaOH/ surf | NaOH/ surf | NaOH/ surf | NaOH/ surf | NaOH | NaOH | NaOH | NaOH / surf |
| comments | | | | | | | | no TiO2 | no TiO2 |
| organic formula ratio | WR/WR control | 1.08 | 5.92 | 0.44 | 0.95 | 0.40 | 0.43 | 1.12 | 1.85 |
| inorganic formula ratio | WR/WR control | 0.40 | 0.33 | 0.98 | 1.65 | 0.79 | 0.75 | 0.87 | 0.54 |

Examples 34-35 - CHA/MMA/MAA polymer nanosilica hybrids prepared from MEK

Examples 34 and 35, as shown in Table 6, illustrate the experimental conditions and water resistance results for polymers of the same composition based on the monomers (cyclohexyl acrylate) CHA, MMA, and MAA, prepared in MEK. The emulsification process was performed either with NaOH alone (as a neutralizer for MAA) or in combination of NaOH and sodium laureth sulfate. The nanosilicas used in these experiments were 10 nm in diameter. As shown in Table 6, the inorganic formulation of Example # 34 exhibits a much better water resistance than the control through our screening protocol.

Table 6

| Example # | | 34 | 35 |
|---|---|---|---|
| SH/nanosilica, wt % | | 3 | 3 |
| emulsification | | NaOH/surf | NaOH |
| IV, lacquer | | 0.35 | 0.35 |
| inorganic formula ratio | WR/WR control | 0.56 | 1.03 |

Examples 36-37 - BA/MMA/MAA/BenzA polymer macro silica hybrids

Examples 36 and 37, as shown in Table 7, illustrate the experimental conditions and water resistance results for polymers of the same composition based on the monomers BA, MMA, MAA and benzyl acrylate (BenzA). The silica used is an amorphous precipitated silica, Lo-Vel™ 6200 available from PPG Industries, Inc., having an average particle size of 8 microns. The ratio "SH/silica, wt%" represents the weight percentage of the silane reagent to the silica on a dry weight basis. The emulsification process performed either with NaOH alone (as a neutralizer for MAA) or in combination of NaOH and sodium laureth sulfate. The organic formulations show an improved water resistance to that of the control through the Water Resistance Screening Test as described hereinabove. The formulations were smooth in appearance.

Table 7

| Example # | | 36 | 37 |
|---|---|---|---|
| SH/silica, wt % | | 2 | 2 |
| emulsification | | NaOH/surf | NaOH |

(continued)

| Example # | | 36 | 37 |
|---|---|---|---|
| organic sunscreen water resistance screening test ratio | WR/WR control | 0.67 | 0.94 |

Example 3 8 - BA/MMA/MAA/BenzA polymer macro silica hybrids using water as a preparation medium

Example 38, as shown in Table 8, illustrates the experimental conditions and water resistance results for polymers of the same composition based on the monomers BA, MMA, MAA and benzyl acrylate (BenzA). The silica used is an amorphous precipitated silica, Lo-Vel™ 6200 available from PPG Industries, Inc., having an average particle size of 8 microns. The ratio "SH/silica, wt%" represents the weight percentage of the silane reagent to the silica on a dry weight basis. In this process, the silica particle was functionalized with the silane reagent utilizing the non-polar monomers (BA/MMA/BenzA) as the medium. This phase was then emulsified in water and polymerized using a standard emulsion polymerization process, adding the remaining monomer (MAA) and the initiating system. All of the emulsifier was present in the initial charge. The final product was thus formed as a suspension of the hybrid product in water. As in previous examples, a base, either organic or inorganic, can be used to stabilize the resulting dispersion.
This product was formulated in the organic sunscreen formulation. It shows an improved water resistance to that of the control through the Water Resistance Screening Test as described hereinabove. The formulation was smooth in appearance.

Table 8

| Example # | | |
|---|---|---|
| SH/silica, wt % | | 2 |
| Stabilizing system | | NaOH/surfactant |
| organic sunscreen water resistance screening test ratio | WR/WR control | 0.89 |

Examples 39-40 - BA/MMA/NaStS polymer macro silica hybrids

Examples 39 and 40 in Table 9, shows the experimental conditions and water resistance results for polymers of the same composition based on the monomers BA, MMA, and sodium styrene sulfonate (NaStS). The silica used is an amorphous precipitated silica, Lo-Vel 6200, of particle size 8 microns. The ratio "SH/silica, wt%" represents the weight percentage of the silane reagent to the silica on a dry weight basis. The emulsification process was done either with NaOH alone (as a neutralizer for MAA) or in combination of NaOH and sodium laureth sulfate. The organic formulation with the hybrid, i.e. Example # 40, shows an improved water resistance to that of the control. All formulations were smooth.

Table 9

| Example # | | 39 | 40 |
|---|---|---|---|
| SH/silica, wt % | | 2 | 2 |
| emulsification | | NaOH/surf | NaOH |
| organic sunscreen water resistance screening test ratio | WR/WR control | 1.69 | 0.59 |

Example 41 - Sunscreen Spray Formulation

A sprayable formulation was prepared by combining 8.56 g of the particle polymer hybrid listed as Example 9 (Table 1) with 88.1 g of ethanol and 3.34 g of the sunscreen octocrylene.

Example 42 - Liquid Foundation

In this example, particles of yellow iron oxide Mapico® High-Purity Yellow Iron Oxide Y50 from Rockwood Pigments were treated with mercaptopropyltrimethoxysilane and subsequently grafted as per the previous examples. The hybrid polymer particle was compared to a system based on the same weight of polymer prepared in the absence of particles, compounded with the same weight of free particles. Example 42-2 represents the liquid formulation including the polymer particle hybrid. Comparative Example 42-1 represents the liquid formulation in the absence of the polymer particle hybrid. The formulations are shown in Table 9.

Table 10

| Liquid Formulation | | | |
|---|---|---|---|
| INCI Name | Trade Name | | |
| | | Examples | |
| Phase A | | **42-1** | **42-2** |
| Ceteareth 20 | Lipocol SC 20 | 4.00 | 4.00 |
| Dicaprylyl Ether | Cetiol OE | 5.00 | 5.00 |
| Ethylhexyl Palmitate | Jeechem OP | 5.00 | 5.00 |
| Ethylhexyl Methoxycinnamate | Neo Heliopan AV | 3.00 | 3.00 |
| Acetylated Lanolin Alcohol | Protolan MOD | 5.00 | 5.00 |
| Mixture | Abil WE09 | 4.00 | 4.00 |
| Dimethicone | DC 200 / 50 cst | 3.00 | 3.00 |
| Benzophenone 3 | Neo Heliopan BB | 5.00 | 5.00 |
| | | | |
| Phase B | | | |
| Water | Water | 44.23 | 40.18 |
| Propylene Glycol | Propylene Glycol | 5.00 | 5.00 |
| control polymer (no particle) | 1.0% Active | 6.29 | |
| | | | |
| Hybrid phase | | | |
| hybrid polymer particle | 1% active on polymer | | 10.52 |
| | | | |
| Phase C | | | |
| TiO2 | Presperse | 6.00 | 6.00 |
| Iron Oxide yellow /E-172 | Presperse | 0.90 | 0.90 |
| iron oxide Y50EC Std | | 0.18 | |
| Iron Oxide Med, Red | preseperse Aquaspersable | 0.55 | 0.55 |
| Iron Oxide jet Black | preseperse Aquaspersable | 0.10 | 0.10 |
| | | | |
| Phase D | | | |
| Cyclopentasiloxane (and | DC 345 | 2.00 | 2.00 |
| cyclohexasiloxane | | | |
| | | | |
| Phase E | | | |
| Preservative | Germall plus liquid | 0.75 | 0.75 |

Procedure for 42-1:

1. Heat Phase A to 75-80C and mix until uniform
2. Heat Phase B to 70-75C.
3. Mix phase B until uniform
4. Premix phase C. Add to Phase B under homogenation. Keep temp at 70-75C
5. Add phase A to phase B under homogenation for 15 minutes. Keep temp at 70-75.
6. Cool to 40C with sweeping agitation
7. Below 40C add D and E . Mix until
uniform.

Procedure for 42-2:

1. Heat Phase A to 75-80C and mix until uniform
2. Heat Phase B to 70-75C.
3. Mix phase B until uniform
4. At 70-75 add the polymer hybrid particle to Phase B under homogenation
5. Premix phase C. Add to Phase B under homogenation. Keep temp at 70-75C
6. Add phase A to phase B under homogenation for 15 minutes. Keep temp at 70-75.
7. Cool to 40C with sweeping agitation
8. Below 40C add D and E. Mix until
uniform.

Formulation 42-2, which was based on non-optimized foundation formula including the polymer particle hybrid system, exhibited a turbidity value of 443 compared to a turbidity value of 786 exhibited by that of the control, Formulation 42-1. Accordingly, the sample comprising the polymer particle hybrid system had a better water resistance in the water screen test than the sample comprising separate additions of particles and polymer.

Example 43 - Eye Liner

This is based on the previous examples, but utilizing an ultramarine blue pigment, NUBICOAT HWR from Nubiola as the particle. These particles are based on silica encapsulates of the pigment, allowing for modification with silanes. The resulting polymer particle hybrid was formulated into an eye liner system as shown in Table 11.

Table 11

| Liquid Eyeliner Formulation Composition with Grafted Iron Oxide (BLUE) | | |
|---|---|---|
| INCI Name | Trade Name | |
| | | Example 43 |
| Phase A | | |
| Water | Water | 47.66 |
| Disodium EDTA | Versene Na2 | 0.10 |
| | | |
| Phase B | | |
| Hydroxyethylcellulose | Natrosol 250 HHR | 0.90 |
| | | |
| Phase C | | |
| Ultramarine Blue | 702191 blue cos ultramarine CG 630 | 2.82 |
| Jet black | Iron oxide | 7.00 |
| polymer particle hybrid (11.1%) | | 10.52 |
| | | |

(continued)

| Phase D | | |
|---|---|---|
| Water | Water | 7.50 |
| | | |
| Phase E | | |
| White Beeswax | White Beeswax | 5.00 |
| Carauba Wax #1 | Carauba Wax #1 | 3.30 |
| PEG 20 Stearate | Jeemate 1000 DPS | 2.00 |
| Glyceryl stearate (and) Laureth 23 | Cerasynt 945 | 3.00 |
| | | |
| | | |
| Phase F | | |
| Preservative | Germall Plus | 0.20 |
| | | |
| Phase G | | |
| Bismuth Oxychloride | Pearl 1 | 7.00 |
| | | |
| TiO2 | TiO2 | 3.00 |
| | | |

1. Heat phase A to 85C
2. Slowly sprinkle in Natrosol. Using homogenizer. Maintain temp.
3. Add C under homogenization and mix until uniform
4. Premix D at RT and add to main beaker. Mix until uniform at 85C
5. Heat E to 87C and add to main beaker.
6. Switch to slow sweep and cool to 45C
7. Add F and G one at a time
8. Sweep to 30C

The resulting formulation was shiny and viscous though pourable. The water resistance screen test gave a turbidity measurement of 12, showing very little disintegration of the formulation when applied to an immersed substrate under dynamic conditions.

**Claims**

1.   A skin care formulation comprising:

   a dispersion or emulsion comprising at least one particle-polymer hybrid wherein the polymer-particle hybrid is formed by functionalizing af least one hydroxyl-containing particle with at least one linking group prior to grafting and grafting at least one polymerizable monomer that is capable of being grafted using free radical polymerization to the functionalized particle; and
   a cosmetically acceptable additive.

2.   The skin care formulation of claims 1 wherein the at least one particle is selected from the group consisting of a $SiO_2$, $ZrO_2$, $TiO_2$, $CeO_2$, ZnO, a clay, a polysaccharide and combinations thereof.

3.   The skin care formulation of claims 1 or 2 Therein the at least one particle has an average particle size in the range of from 10 nm to 1 micron, preferably from 100 nm to 1 micron.

4. The skin care formulation of claim 1 or 2 wherein the at least one particle has an average particle size in the range of from 1 micron or greater up to 500 microns.

5. The skin care formulation of any one of the preceding claims wherein the at least one particle is an aggregate of particles or an agglomerate of particles.

6. The skin care formulation of any one of the preceding claims wherein the polymer comprises at least one monomer that is an acrylate, a methacrylate or a styrene or derivatives thereof.

7. The skin care formulation of any one of the preceding claims wherein the at least one polymer comprises at least one monomer selected from the group consisting of butyl acrylate, methyl methacrylate, cyclohexyl acrylate, benzyl acrylate, acrylic acid, methacrylic acid, and sodium styrene sulfonate and combinations thereof.

8. The skin care formulation of any one of the preceding claims wherein the particles are at least partially functionalized with a linking group comprising an organofunctional silane, preferably vinyl- or thiol- functional silanes or non-terminal disulfide groups.

9. The skin care formulation of any one of the preceding claims wherein the functionalized particles are obtained by reaction of a mercaptopropyltrialkoxysilane on the particle, preferably a mercaptopropyltrimethoxysilane or mercaptopropyltriethoxysilane.

10. The skin care formulation of any one of the preceding claims wherein the intrinsic viscosity, IV, of the emulsion or dispersion is in the range of from 0.1 to 2.

11. The skin care formulation of any one of the preceding claims wherein the dispersion or emulsion is present in an amount of 1% to 25% of the skin care formulation.

12. The skin care formulation of any one of the preceding claims wherein the skin care formulation is a spray-on, rub-on, spread-on, mousse, oil-based, alcohol-based, or solid (stick) formulation.

13. The skin care formulation of any one of the preceding claims wherein the skin care formulation further comprises a stabilizing polymer or a waterproofing polymer.

14. The skin care formulation of any one of the preceding claims wherein the at least one particle is grafted with one polymer

15. A method of preparing the skin care formulation of any one of claims 1-14 comprising:

polymerizing at least one partially functionalized particle with at least one monomer in the presence of a solvent or diluent;
mixing the resulting grafted particle-polymer hybrid with a cosmetically acceptable additive; and
optionally converting the grafted polymer-particle hybrid to an aqueous system.

**Patentansprüche**

1. Hautpflegeformulierung mit:

einer Dispersion oder Emulsion mit mindestens einem Partikel-Polymer-Hybrid, wobei das Polymer-Partikel-Hybrid durch Funktionalisieren von mindestens einem hydroxylhaltigen Partikel mit mindestens einer Verkettungsgruppe vor dem Pfropfen und Pfropfen von mindestens einem polymerisierbaren Monomer gebildet wird, das unter Einsatz einer radikalischen Polymerisation auf das funktionalisierte Partikel aufgepfropft werden kann; sowie
einem kosmetisch zulässigen Zusatz.

2. Hautpflegeformulierung nach Anspruch 1, wobei das mindestens eine Partikel aus der Gruppe bestehend aus einem $SiO_2$, $ZrO_2$, $TiO_2$, $CeO_2$, ZnO, einem Ton, einem Polysaccharid sowie Kombinationen aus diesen ausgewählt ist.

3. Hautpflegeformulierung nach Anspruch 1 oder 2, wobei das mindestens eine Partikel eine durchschnittliche Partikelgröße im Bereich von 10 nm bis 1 Mikrometer, vorzugsweise von 100 nm bis 1 Mikrometer, aufweist.

4. Hautpflegeformulierung nach Anspruch 1 oder 2, wobei das mindestens eine Partikel eine durchschnittliche Partikelgröße im Bereich von 1 Mikrometer oder größer bis zu 500 Mikrometer aufweist.

5. Hautpflegeformulierung nach einem der vorhergehenden Ansprüche, wobei das mindestens eine Partikel eine Anhäufung von Partikeln oder ein Agglomerat von Partikeln ist.

6. Hautpflegeformulierung nach einem der vorhergehenden Ansprüche, wobei das Polymer mindestens ein Monomer umfasst, das aus einem Acrylat, einem Methacrylat oder einem Styrol oder Derivaten von diesen besteht.

7. Hautpflegeformulierung nach einem der vorhergehenden Ansprüche, wobei das mindestens eine Polymer mindestens ein Monomer ausgewählt aus der Gruppe bestehend aus Butylacrylat, Methylmethacrylat, Cyclohexylacrylat, Benzylacrylat, Acrylsäure, Methacrylsäure und Natriumstyrolsulfonat sowie Kombinationen aus diesen umfasst.

8. Hautpflegeformulierung nach einem der vorhergehenden Ansprüche, wobei die Partikel zumindest teilweise mit einer Verkettungsgruppe funktionalisiert sind, welche ein organofunktionelles Silan, vorzugsweise vinyl- oder thiolfunktionelle Silane oder nicht endständige Disulfidgruppen, umfasst.

9. Hautpflegeformulierung nach einem der vorhergehenden Ansprüche, wobei die funktionalisierten Partikel durch Reaktion eines Mercaptopropyltrialkoxysilans auf dem Partikel, vorzugsweise eines Mercaptopropyltrimethoxysilans oder Mercaptopropyltriethoxysilans, erhalten werden.

10. Hautpflegeformulierung nach einem der vorhergehenden Ansprüche, wobei die Eigenviskosität, IV, der Emulsion oder Dispersion im Bereich von 0,1 bis 2 liegt.

11. Hautpflegeformulierung nach einem der vorhergehenden Ansprüche, wobei die Dispersion oder Emulsion in einer Menge von 1% bis 25% der Hautpflegeformulierung vorliegt.

12. Hautpflegeformulierung nach einem der vorhergehenden Ansprüche, wobei die Hautpflegeformulierung aus einer Formulierung zum Aufsprühen, zum Einreiben, zum Verteilen, in Schaumform, auf Ölbasis, auf Alkoholbasis oder als Feststoff(-Stift) besteht.

13. Hautpflegeformulierung nach einem der vorhergehenden Ansprüche, wobei die Hautpflegeformulierung weiterhin ein stabilisierendes Polymer oder ein imprägnierendes Polymer umfasst.

14. Hautpflegeformulierung nach einem der vorhergehenden Ansprüche, wobei das mindestens eine Partikel mit einem Polymer gepfropft ist.

15. Verfahren zur Herstellung der Hautpflegeformulierung nach einem der Ansprüche 1-14, mit den Schritten:

Polymerisieren von mindestens einem teilweise funktionalisierten Partikel mit mindestens einem Monomer in Anwesenheit eines Lösungsmittels oder Verdünnungsmittels;
Vermischen des daraus resultierenden gepfropften Partikel-Polymer-Hybrids mit einem kosmetisch zulässigen Zusatz; und
wahlweise Umwandeln des gepfropften Polymer-Partikel-Hybrids in ein wässriges System.

**Revendications**

1. Formulation de soins de la peau comprenant :

une dispersion ou une émulsion comprenant au moins un hybride de particule-polymère dans laquelle l'hybride de polymère-particules est formé en fonctionnalisant au moins une particule contenant un groupe hydroxyle avec au moins un groupe de liaison avant le greffage et en greffant au moins un monomère polymérisable qui est capable d'être greffé en utilisant la polymérisation par radicaux libres sur la particule fonctionnalisée ; et un additif cosmétiquement acceptable.

**2.** Formulation de soins de la peau selon la revendication 1, dans laquelle l'au moins une particule est choisie dans le groupe constitué de $SiO_2$, $ZrO_2$, $TiO_2$, $CeO_2$, ZnO, d'une argile, d'un polysaccharide et de leurs combinaisons.

**3.** Formulation de soins de la peau selon la revendication 1 ou 2, dans laquelle l'au moins une particule a une taille de particule moyenne se trouvant dans la plage allant de 10 nm à 1 micron, de préférence allant de 100 nm à 1 micron.

**4.** Formulation de soins de la peau selon la revendication 1 ou 2, dans laquelle l'au moins une particule a une taille de particule moyenne se trouvant dans la plage allant de 1 micron jusqu'à 500 microns.

**5.** Formulation de soins de la peau selon l'une quelconque des revendications précédentes, dans laquelle l'au moins une particule est un agrégat de particules ou un agglomérat de particules.

**6.** Formulation de soins de la peau selon l'une quelconque des revendications précédentes, dans laquelle le polymère comprend au moins un monomère qui est un acrylate, un méthacrylate ou un styrène ou leurs dérivés.

**7.** Formulation de soins de la peau selon l'une quelconque des revendications précédentes, dans laquelle l'au moins un polymère comprend au moins un monomère choisi dans le groupe constitué d'acrylate de butyle, de méthacrylate de méthyle, d'acrylate de cyclohexyle, d'acrylate de benzyle, d'acide acrylique, d'acide méthacrylique et de styrène sulfonate de sodium et leurs combinaisons.

**8.** Formulation de soins de la peau selon l'une quelconque des revendications précédentes, dans laquelle les particules sont au moins partiellement fonctionnalisées avec un groupe de liaison comprenant un silane organofonctionnel, de préférence des silanes à fonction vinyle ou à fonction thiol ou des groupes bisulfure non terminaux.

**9.** Formulation de soins de la peau selon l'une quelconque des revendications précédentes, dans laquelle les particules fonctionnalisées sont obtenues par réaction d'un mercaptopropyltrialcoxysilane sur la particule, de préférence un mercaptopropyltriméthoxysilane ou un mercaptopropyltriéthoxysilane.

**10.** Formulation de soins de la peau selon l'une quelconque des revendications précédentes, dans laquelle la viscosité intrinsèque, IV, de l'émulsion ou de la dispersion se trouve dans la plage allant de 0,1 à 2.

**11.** Formulation de soins de la peau selon l'une quelconque des revendications précédentes, dans laquelle la dispersion ou l'émulsion est présente en une quantité allant de 1% à 25% de la formulation de soins de la peau.

**12.** Formulation de soins de la peau selon l'une quelconque des revendications précédentes, dans laquelle la formulation de soins de la peau est une formulation pouvant être pulvérisée, une formulation pouvant être frottée, une formulation pouvant être étalée, une formulation en mousse, une formulation à base d'huile, une formulation à base d'alcool ou une formulation solide (bâton).

**13.** Formulation de soins de la peau selon l'une quelconque des revendications précédentes, dans laquelle la formulation de soins de la peau comprend en outre un polymère stabilisant ou un polymère imperméable à l'eau.

**14.** Formulation de soins de la peau selon l'une quelconque des revendications précédentes, dans laquelle l'au moins une particule est greffée avec un polymère.

**15.** Procédé de préparation de la formulation de soins de la peau selon l'une quelconque des revendications 1 à 14 comprenant :

la polymérisation d'au moins une particule partiellement fonctionnalisée avec au moins un monomère en présence d'un solvant ou d'un diluant ;
le mélange de l'hybride de polymère-particules résultant avec un additif cosmétiquement acceptable ; et
l'éventuelle transformation de l'hybride de polymère-particules greffé en un système aqueux.

**EP 2 694 015 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009086260 A
- WO 2007095324 A
- WO 2009131910 A
- US 20100278764 A
- US 6482397 B
- US 6261541 B
- US 6231837 B
- US 6488916 B
- US 20090035234 A

**Non-patent literature cited in the description**

- The CTFA Cosmetic Ingredient Handbook. 1997
- THE CTFA COSMETIC INGREDIENT HANDBOOK. 2002, XV